# EUROPEAN PATENT SPECIFICATION

(11) **EP 4 310 086 B1**
(45) Date of publication and mention of the grant of the patent: **01.07.2026**
(21) Application number: 22771770.9
(22) Date of filing: 16.03.2022
(51) Int. Cl.: C07D 471/04, A61K 31/437, A61P 25/28, A61P 29/00, A61P 37/00

(54) **CRYSTALLINE FORM OF N-(4-(1-(2-CYANOACETYL)-3-METHYL-1,2,3,6-TETRAHYDROPYRIDIN-4-YL)-1H-PYRROLO[2,3-B]PYRIDIN-6-YL)CYCLOPROPANECARBOXAMIDE**
KRISTALLINE FORM VON N-(4-(1-(2-CYANOACETYL)-3-METHYL-1,2,3,6-TETRAHYDROPYRIDIN-4-YL)-1H-PYRROLO[2,3-B]PYRIDIN-6-YL)CYCLOPROPANCARBOXAMID
FORME CRISTALLINE DU N-(4-(1-(2-CYANOACÉTYL)-3-MÉTHYL-1,2,3,6-TÉTRAHYDROPYRIDIN-4-YL)-1H-PYRROLO[2,3-B]PYRIDIN-6-YL)CYCLOPROPANECARBOXAMIDE

(30) Priority: 16.03.2021 KR 20210034296
(43) Date of publication of application: 24.01.2024
(73) Proprietor: HK inno.N Corporation, Cheongju-si, Chungcheongbuk-do 28158 (KR)
(72) Inventor: KIM, Young Ju, Seoul 04551 (KR); KIM, Jun, Seoul 04551 (KR); KIM, Hanna, Seoul 04551 (KR); KWEON, Jae Hong, Seoul 04551 (KR); LEE, Ye-Lim, Seoul 04551 (KR); HWANG, Do Seok, Seoul 04551 (KR)
(74) Representative: HGF
(86) International application number: PCT/KR2022/003703
(87) International publication number: WO 2022/197104

(56) References cited:
- KR-A- 20140 004 637
- KR-A- 20140 027 318
- KR-A- 20190 039 823
- KR-A- 20190 043 437
- KR-A- 20190 068 626

## Description

### Technical Field

The present invention relates to a novel crystalline form of a heterocyclic compound as a protein kinase inhibitor and a preparation method thereof.

### Background

In general, it is obvious that the same drug may show a difference in pharmaceutically important properties such as solubility or dissolution properties and bioavailability in each form such as a noncrystalline form, one or more crystalline forms, salts, and the like. In selecting the noncrystalline and crystalline forms, the noncrystalline form has high solubility, and thus has advantages in increasing drug efficacy and exhibiting a rapid action, but has disadvantages in being unstable, having a short shelf life, and having difficulty to control a release rate and a blood concentration of a drug. On the contrary, the crystalline form has low solubility and thus has low bioavailability per unit weight, but has an advantage in preparing a formulation capable of securing stability and continuous release. As such, since the crystalline form is stable, but has low solubility compared to the noncrystalline form, the solubility needs to be sacrificed when preferentially considering stability. On the contrary, there is a dilemma in which the stability needs to be sacrificed when preferentially considering the solubility, and thus it is very difficult to obtain a crystal which satisfies both stability and solubility at the same time.

Janus kinase (JAK) is an enzyme which controls various intracellular processes by phosphorylating other proteins to regulate the activity, position, and function of the proteins. The Janus kinase is located at an intracellular receptor of an inflammatory cytokine, and the inflammatory cytokine binds with the receptor, phosphorylates, and transmits a signal of the inflammatory cytokine into cells through an action with STAT molecules. An excessive activation of signal transduction through such various inflammatory cytokines causes an immune system of our body to attack the body and thus leads to the occurrence of autoimmune diseases. In recent years, it has been reported that JAK1 inhibitors rapidly ameliorate the severity and symptoms of Alzheimer's disease in phase II and phase III clinical trials on selective JAK1 inhibitors, upadasitinib and abrocitinib.

### Detailed Description of the Invention

### Technical Problem

One object of the present invention is to provide a novel crystalline form of a heterocyclic compound as a protein kinase inhibitor.

Other object of the present invention is to provide a method for preparing the crystalline form.

### Technical Solution

The inventors of the present application have made efforts to discover a compound having improved physicochemical properties, capable of minimizing the occurrence of related materials by enhancing stability against heat and moisture, while having a pharmacological activity equal to or higher than that of an existing compound, and thus have identified a crystalline form of a heterocyclic compound according to the present invention, thereby completing the present invention.

In the crystalline form of the heterocyclic compound as a protein kinase inhibitor according to the present invention, the heterocyclic compound is represented by formula I below.

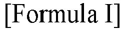

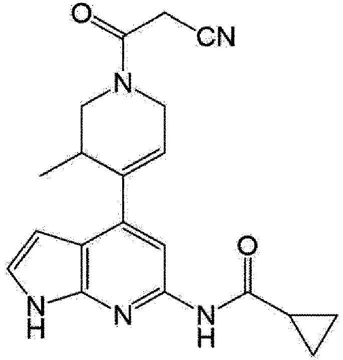

The name of the heterocyclic compound represented by above formula I is N-(4-(1-(2-cyanoacetyl)-3-methyl-1,2,3,6-tetrahydropyridin-4-yl)-1H-pyrrolo[2,3-b]pyridine-6-yl)cyclopropanecarboxamide.

The heterocyclic compound represented by above formula I may be represented by each of (S)-N-(4-(1-(2-cyanoacetyl)-3-methyl-1,2,3,6-tetrahydropyridin-4-yl)-1H-pyrrolo[2,3-b]pyridine-6-yl)cyclopropanecarboxamide and (R)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, or may be a mixture thereof.

In one embodiment, the heterocyclic compound of the present invention or pharmaceutically acceptable salts thereof may be a compound represented by formula II below or pharmaceutically acceptable salts thereof.

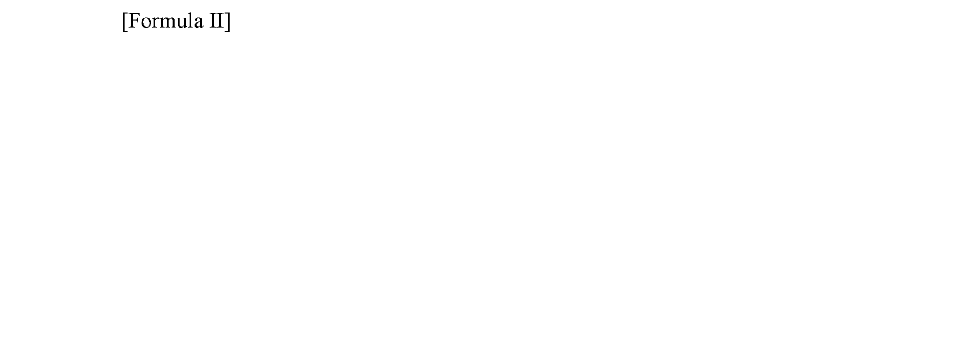

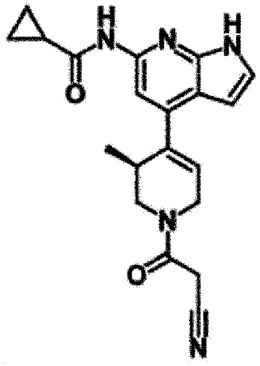

The name of the heterocyclic compound represented by above formula II is (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide.

A powder X-ray diffraction (XRD) pattern of the crystalline form according to the present invention includes diffraction peaks at diffraction angle 2θ (±0.2°) values of 4.6°, 8.1°, and 11.2°.

The powder X-ray diffraction (XRD) pattern may further include at least one of diffraction peaks at diffraction angle 2θ (±0.2°) values of 8.8°, 15.5°, and 20.3°.

The powder X-ray diffraction (XRD) pattern includes diffraction peaks at diffraction angle 2θ (±0.2°) values of 4.6°, 8.1°, 8.8°, 11.2°, 15.5° and 20.3°.

In one embodiment, the powder X-ray diffraction (XRD) pattern may include diffraction peaks at diffraction angle 2θ (±0.2°) values of 4.6°, 8.1°, 8.8°, 11.2°, 12.1°, 15.5°, 20.3° and 22.4°.

The crystalline form according to the present invention has a mass reduction rate of 1.6% at 150°C as a result of thermogravimetric analysis (TGA).

The crystalline form according to the present invention shows a differential scanning calorimetry (DSC) endothermic peak at 142.07 to 157.29°C. In this case, the differential scanning calorimetry endothermic peak may appear when a temperature rising rate is 10°C/min.
**(1)** There may be provided a crystalline form of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, in which the X-ray powder diffraction pattern includes diffraction peaks at diffraction angle 2θ (±0.2°) values of 4.6°, 8.1° and 11.2°.
**(2)** There may be provided the crystalline form according to above **(1),** in which the X-ray powder diffraction pattern further includes at least one of diffraction peaks at diffraction angle 2θ (±0.2°) values of 8.8°, 15.5°, and 20.3°.
**(3)** There may be provided the crystalline form according to above **(1)** or **(2),** in which the X-ray powder diffraction pattern includes diffraction peaks at diffraction angle 2θ (±0.2°) values of 4.6°, 8.1°, 8.8° and 11.2°.
**(4)** There may be provided the crystalline form according to above **(1), (2)** or **(3),** in which the crystalline form includes a mass reduction rate of 1.6% at 150°C as a result of thermogravimetric analysis (TGA).
**(5)** There may be provided the crystalline form according to above **(1), (2), (3)** or **(4),** in which the crystalline form shows a differential scanning calorimetry (DSC) endothermic peak at 142.07 to 157.29°C.

The crystalline form of the heterocyclic compound according to the present invention may be prepared by the following method, which includes:
(A) mixing N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, which is a heterocyclic compound represented by formula I according to the present invention, with an organic solvent, water or a mixture thereof; and
(B) stirring the resulting product obtained in above (A) to precipitate crystals.

The resulting product obtained in above (A) may be a solution in which N-(4-(1-(2-cyanoacetyl)-3-methyl-1,2,3,6-tetrahydropyridin-4-yl)-1H-pyrrolo[2,3-b]pyridin-6-yl)cyclopropanecarboxamide is dissolved in an organic solvent, water or a mixture thereof, or a suspension in which N-(4-(1-(2-cyanoacetyl)-3-methyl-1,2,3,6-tetrahydropyridin-4-yl)-1H-pyrrolo[2,3-b]pyridin-6-yl)cyclopropanecarboxamide is suspended in an organic solvent, water or a mixture thereof.

The organic solvent used in above (A) may be ethyl acetate, ethyl formate, dichloromethane, acetone, methanol, ethanol, isopropanol, acetonitrile, toluene, tert-butyl methyl ether, 2-butanone, or a mixture thereof.

Above (B) may be performed to cool down or heat the mixed solution to be stirred.

The preparation method may further include (C) adding an anti-solvent to mature crystals, after the precipitating of the crystals of above (B). In this case, the anti-solvent may be water, hexane, heptane, tert-butyl methyl ether, isopropyl ether, cyclohexane, or a mixture thereof.

### Advantageous Effects

A crystalline form of the heterocyclic compound represented by formula I according to the present invention has excellent physical stability and thus can be advantageously used in formulating medicines.

### Brief Description of the Drawings

FIG. 1 is a graph showing the results of XRPD analysis before and after exposure of the crystalline form of the present invention to stress conditions.
FIG. 2 is a view showing the results of DSC analysis on the crystalline form of the present invention.
FIG. 3 is a view showing the results of TGMS analysis on the crystalline form of the present invention.

### Best Mode for Invention

Hereinafter, the present invention will be described with reference to examples. All the terms used herein including technical or scientific terms have the same meaning as commonly understood by those ordinary skilled in the art, to which the present invention pertains, unless defined otherwise. Such terms as those defined in a generally used dictionary are to be interpreted to have the meanings equal to the contextual meanings in the relevant art, and are not to be interpreted to have ideal or excessively formal meanings, unless clearly defined in the present application.

### Preparing Example 1: Synthesis of (S)-N-(4-(1-(2-cyanoacetyl)-3-methyl-1,2,3,6-tetrahydropyridin-4-yl)-1H-pyrrolo[2,3-b]pyridin-6-yl)cyclopropanecarboxamide

The title compound was prepared according to the method disclosed in Korean Unexamined Patent Application No. 2019-0043437.

¹H NMR (400 MHz, DMSO-d₆) δ 11.44 (s, 1H), 10.57 (s, 1H), 7.84 (d, J = 10.2 Hz, 1H), 7.34 (d, J = 3.1 Hz, 1H), 6.48 (dd, J = 1.8, 3.7 Hz, 1H), 6.17 - 6.03 (m, 1H), 4.31 - 4.01 (m, 6H), 3.96 - 3.62 (m, 2H), 3.02 (m J = 36.6 Hz, 1H), 2.02 (s, 1H), 0.88 (s, 3H), 0.84 - 0.73 (m, 4H); MS(ESI+) m/z 364 (M+H)⁺

### Example 1: Preparation of crystalline form A

32 mg of the compound according to formula II obtained in Preparation Example 1 was mixed with 100 mL of ethyl formate to obtain a suspension. The suspension was continuously stirred at room temperature for 24 hours, centrifuged to obtain a solid, and vacuum-dried (30°C, 10 mbar) to obtain a solid product.

### Example 2: Preparation of crystalline form A

In addition, 32 mg of the compound according to formula II obtained in Preparation Example 1 was mixed with 100 mL of acetonitrile to obtain a suspension. The suspension was continuously stirred at room temperature for 24 hours, centrifuged to obtain a solid, and vacuum-dried (30°C, 10 mbar) to obtain a solid product.

### Analysis and measurement method

### 1. XRPD analysis

A plate for obtaining an XRPD pattern was mounted on a Bruker General Area Detector Diffraction System (GADDS) equipped with a VÅNTEC-500 gas domain detector calibrated for intensity and geometric changes. Calibration of measurement accuracy (peak position) was performed using NIST SRM1976 standard (Corundum). Data collection was performed at room temperature using monochromatic CuKα radiation in a diffraction angle (2θ) region of 1.5° to 41.5°, the most distinct part of the XRPD pattern. The diffraction pattern of each well was collected at two 2θ ranges (1.5° ≤ 2θ ≤ 21.5° for a first frame and 9.5° ≤ 2θ ≤ 41.5° for a second frame) with an exposure time of 90 seconds for each frame. No background subtraction or curve smoothing was applied to the XRPD pattern. The carrier material used in the XRPD analysis was transparent to X-rays.

### 2. DSC analysis

Melt properties were obtained from DSC thermograms recorded with a heat flux DSC822e apparatus (product name, Mettler-Toledo GmbH, Switzerland). DSC822e corrected temperature and enthalpy with a small indium piece (melting point at 156.6°C; ΔHf=28.45 J/g). A sample was sealed in a standard 40 µL aluminum pan, followed by drilling a pin-hole therein, and then heated from 25°C to 300°C in a DSC at a heating rate of 10°C/min. Dry N₂ gas was used at a flow rate of 50 mL/min to purge a DSC equipment during the measurement.

### 3. TGA/SDTA and TGMS analysis

A mass loss caused by solvent or a moisture loss from crystals was determined by thermogravimetric analysis/simultaneous differential thermal analysis (TGA/SDTA). A weight versus temperature curve was generated by monitoring the weight of samples during heating in a TGA/SDTA851e apparatus (product name, Mettler-Toledo GmbH, Switzerland). TGA/SDTA851e was calibrated with indium and aluminum samples. The sample was placed in a 100 µL aluminum crucible and sealed, and then a pin-hole was drilled therein. The crucible was heated from 25°C to 300°C in TGA at a heating rate of 10°C/min. Dry N₂ gas was used for purging. The gas coming out of the TGA sample was analyzed by Omnistar GSD 301 T2 (product name, Pfeiffer Vacuum GmbH, Germany), which was a quadrupole mass spectrometer for analyzing a mass in the range of 0-200 amu.

### 4. Experiment on physical stability evaluation

Samples were packaged in the form of ((LPDE+N2)+silica gel 1g+LDPE)+Al-Bag, and stored under stress conditions (60°C±2°C/80% RH±5%) for two days before evaluation.

### Analysis/measurement/evaluation results

### 1. Results of XPRD analysis and stability evaluation on crystalline form

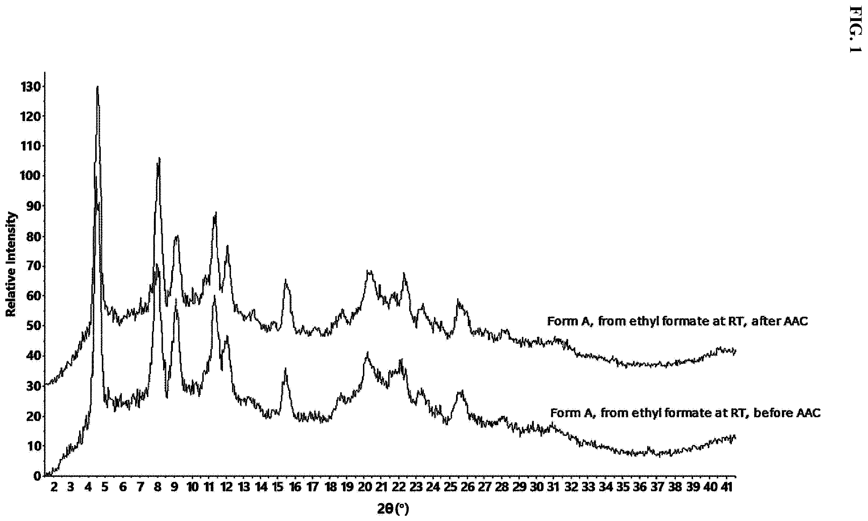

The crystalline forms obtained according to Examples 1 and 2 were subjected to an XPRD analysis method as described above to obtain a XPRD analysis graph. The results thereof were shown in FIG. 1. As a result of XPRD obtained in FIG. 1, diffraction angles are shown in Table 1 below.

**[Table 1]**

| **Diffraction angle (2θ, unit: °)** | **Relative intensity** |
|---|---|
| 4.6 | Strong |
| 8.1 | Strong |
| 8.8 | Moderate |
| 11.2 | Strong |
| 12.1 | Moderate |
| 15.5 | Moderate |
| 20.3 | Moderate |
| 22.4 | Moderate |

Referring to FIG. 1 and Table 1, it can be confirmed that the crystalline forms obtained according to Examples 1 and 2 show diffraction peaks having dominant intensity at specific diffraction angles and accordingly, it is proved that they are not amorphous but crystalline forms.

In particular, referring to FIG. 1, it can be confirmed that even when the crystalline forms obtained according to Examples 1 and 2 are placed under stress conditions, there is substantially no change in the XPRD pattern before/after the stress conditions. Accordingly, it can be seen that the crystalline form according to the present invention has excellent physical stability and thus long-term storage stability is secured.

### 2. Results of DSC analysis on crystalline form

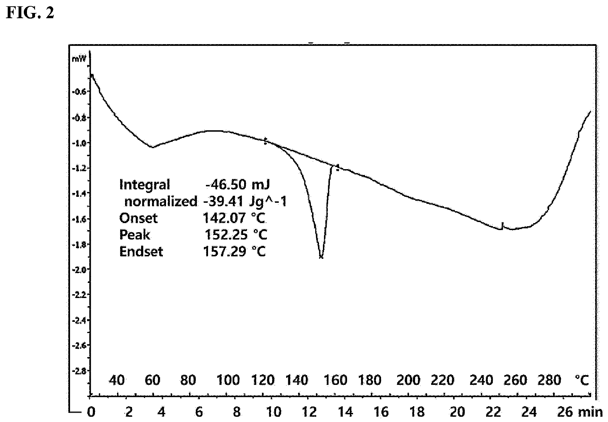

The crystalline forms obtained according to Examples 1 and 2 were subjected to a DSC analysis method as described above to obtain a DSC analysis graph. The results thereof were shown in FIG. 2.

Referring to FIG. 2, the crystalline form according to the present invention shows a differential scanning calorimetry (DSC) endothermic peak at 142.07 to 157.29°C.

### 3. Results of TGMS analysis on crystalline form

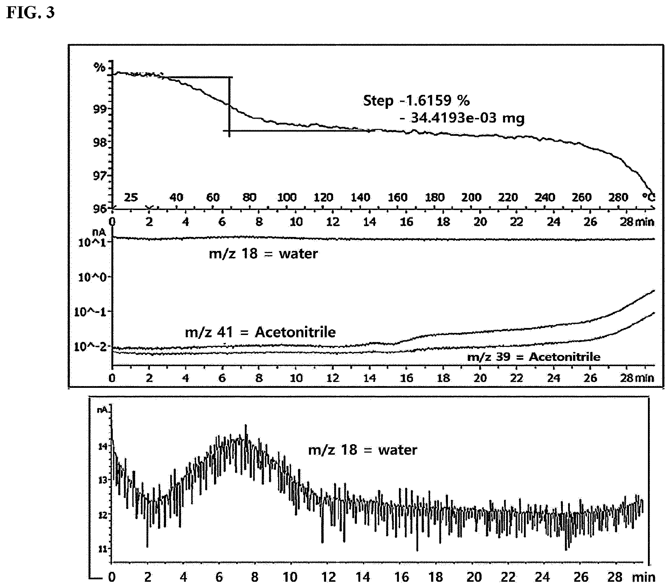

The crystalline forms obtained according to Examples 1 and 2 were subjected to a TGA/SDTA and TGMS analysis method as described above to obtain a TGMS analysis graph. The results thereof were shown in FIG. 3.

Referring to FIG. 3, as a result of thermogravimetric analysis (TGA), it can be confirmed that the crystalline form of the present invention has a mass reduction rate of 1.6% at 150°C. It can be confirmed that the crystalline form of the present invention starts thermal decomposition at about 260°C.

In addition, it can be confirmed that the crystalline form of the present invention is a non-solvated form and an anhydrous form.

## Claims

1. A crystalline form of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide, wherein an X-ray powder diffraction pattern comprises diffraction peaks at diffraction angle 2θ (±0.2°) values of 4.6°, 8.1° and 11.2°.

2. The crystalline form of claim 1, wherein the X-ray powder diffraction pattern further comprises at least one of diffraction peaks at diffraction angle 2θ (±0.2°) values of 8.8°, 15.5° and 20.3°.

3. The crystalline form of claim 1, wherein the X-ray powder diffraction pattern comprises diffraction peaks at diffraction angle 2θ (±0.2°) values of 4.6°, 8.1°, 8.8° and 11.2°.

4. The crystalline form of claim 1, wherein the crystalline form comprises a mass reduction rate of 1.6% at 150°C as a result of thermogravimetric analysis (TGA).

5. The crystalline form of claim 1, wherein the crystalline form shows a differential scanning calorimetry (DSC) endothermic peak at 142.07 to 157.29°C.

6. A method for preparing a crystalline form of N-(4-(1-(2-cyanoacetyl)-3-methyl-1,2,3,6-tetrahydropyridin-4-yl)-1H-pyrrolo[2,3-b]pyridin-6-yl)cyclopropanecarboxamide according to claim 1, the method comprising:
(A) mixing N-(4-(1-(2-cyanoacetyl)-3-methyl-1,2,3,6-tetrahydropyridin-4-yl)-1H-pyrrolo[2,3-b]pyridin-6-yl)cyclopropanecarboxamide with an organic solvent, water or a mixture thereof; and
(B) stirring a resulting product obtained in above (A) to precipitate crystals.

7. The method of claim 6, wherein the resulting product obtained in above (A) is a solution in which N-(4-(1-(2-cyanoacetyl)-3-methyl-1,2,3,6-tetrahydropyridin-4-yl)-1H-pyrrolo[2,3-b]pyridin-6-yl)cyclopropanecarboxamide is dissolved, or a suspension in which N-(4-(1-(2-cyanoacetyl)-3-methyl-1,2,3,6-tetrahydropyridin-4-yl)-1H-pyrrolo[2,3-b]pyridin-6-yl)cyclopropanecarboxamide is suspended.

8. The method of claim 6, wherein the organic solvent is ethyl acetate, ethyl formate, dichloromethane, acetone, methanol, ethanol, isopropanol, acetonitrile, toluene, tert-butyl methyl ether, 2-butanone, or a mixture thereof.

## Patentansprüche

1. Kristalline Form von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid, wobei ein Röntgenpulverbeugungsmuster Beugungspeaks bei Werten für einen Beugungswinkel 20 (±0,2°) von 4,6°, 8,1° und 11,2° umfasst.

2. Kristalline Form nach Anspruch 1, wobei das Röntgenpulverbeugungsmuster ferner mindestens einen von Beugungspeaks bei Werten für einen Beugungswinkel 20 (±0,2°) von 8,8°, 15,5° und 20,3° umfasst.

3. Kristalline Form nach Anspruch 1, wobei das Röntgenpulverbeugungsmuster Beugungspeaks bei Werten für einen Beugungswinkel 20 (±0,2°) von 4,6°, 8,1°, 8,8° und 11,2° umfasst.

4. Kristalline Form nach Anspruch 1, wobei die kristalline Form als Ergebnis einer thermogravimetrischen Analyse (TGA) eine Massenreduzierungsrate von 1,6 % bei 150 °C umfasst.

5. Kristalline Form nach Anspruch 1, wobei die kristalline Form einen endothermen Peak der Differentialscanning-Kalorimetrie (DSC) bei 142,07 bis 157,29 °C zeigt.

6. Verfahren zur Herstellung einer kristallinen Form von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid nach Anspruch 1, wobei das Verfahren Folgendes umfasst:
(A) Mischen von N-(4-(1-(2-Cyanoacetyl)-3-methyl-1,2,3,6-tetrahydropyridin-4-yl)-1H-pyrrolo[2,3-b]pyridin-6-yl)cyclopropancarboxamid mit einem organischen Lösungsmittel, Wasser oder einer Mischung davon; und
(B) Rühren eines resultierenden Produkts, das in (A) oben erhalten wurde, um Kristalle auszufällen.

7. Verfahren nach Anspruch 6, wobei das resultierende Produkt, das in (A) oben erhalten wurde, eine Lösung ist, in der N-(4-(1-(2-Cyanoacetyl)-3-methyl-1,2,3,6-tetrahydropyridin-4-yl)-1H-pyrrolo[2,3-b]pyridin-6-yl)cyclopropancarboxamid gelöst ist, oder eine Suspension ist, in der N-(4-(1-(2-Cyanoacetyl)-3-methyl-1,2,3,6-tetrahydropyridin-4-yl)-1H-pyrrolo[2,3-b]pyridin-6-yl)cyclopropancarboxamid suspendiert ist.

8. Verfahren nach Anspruch 6, wobei das organische Lösungsmittel Ethylacetat, Ethylformiat, Dichlormethan, Aceton, Methanol, Ethanol, Isopropanol, Acetonitril, Toluol, tert-Butylmethylether, 2-Butanon oder eine Mischung davon ist.

## Revendications

1. Forme cristalline de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide, dans laquelle un motif de diffraction des rayons X sur poudre comprend des pics de diffraction à des valeurs d'angle de diffraction 20 (±0,2°) de 4,6°, 8,1° et 11,2°.

2. Forme cristalline de la revendication 1, dans laquelle le motif de diffraction des rayons X sur poudre comprend en outre au moins l'un des pics de diffraction à des valeurs d'angle de diffraction 20 (±0,2°) de 8,8°, 15,5° et 20,3°.

3. Forme cristalline de la revendication 1, dans laquelle le motif de diffraction des rayons X sur poudre comprend des pics de diffraction à des valeurs d'angle de diffraction 2θ (±0,2°) de 4,6°, 8,1°, 8,8° et 11,2°.

4. Forme cristalline de la revendication 1, dans laquelle la forme cristalline comprend un taux de réduction de masse de 1,6 % à 150 °C à la suite d'une analyse thermogravimétrique (ATG).

5. Forme cristalline de la revendication 1, dans laquelle la forme cristalline présente un pic endothermique d'analyse calorimétrique différentielle (DSC) entre 142,07 et 157,29 °C.

6. Procédé permettant la préparation d'une forme cristalline de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide selon la revendication 1, le procédé comprenant :
(A) le mélange de N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide avec un solvant organique, de l'eau ou un mélange de ceux-ci ; et
(B) l'agitation d'un produit résultant obtenu en (A) ci-dessus pour précipiter les cristaux.

7. Procédé de la revendication 6, dans lequel le produit résultant obtenu en (A) ci-dessus est une solution dans laquelle le N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide est dissous, ou une suspension dans laquelle le N-(4-(1-(2-cyanoacétyl)-3-méthyl-1,2,3,6-tétrahydropyridin-4-yl)-1H-pyrrolo[2,3-b]pyridin-6-yl)cyclopropanecarboxamide est en suspension.

8. Procédé de la revendication 6, dans lequel le solvant organique est l'acétate d'éthyle, le formiate d'éthyle, le dichlorométhane, l'acétone, le méthanol, l'éthanol, l'isopropanol, l'acétonitrile, le toluène, l'éther méthylique de tert-butyle, la 2-butanone, ou un mélange de ceux-ci.
